# EUROPEAN PATENT APPLICATION

(11) **EP 1 486 167 A1**
(43) Date of publication of application: **15.12.2004**
(21) Application number: 03013056.1
(22) Date of filing: 10.06.2003
(51) Int. Cl.: A61B 5/05, A61B 5/024, G04G 1/00

(54) **wrist watch with heartbeat and body fat measuring mechanism**

(71) Applicant: Kue-Kiong, Thong, Chungli City, Taoyuan Hsien (TW)
(72) Inventor: Kue-Kiong, Thong, Chungli City, Taoyuan Hsien (TW)
(74) Representative: Sroka, Peter-Christian, Dipl.-Ing.

(57) **Abstract**

A wrist watch with heartbeat and body fat measuring mechanism mainly includes a watch body (1) being externally provided with a liquid crystal display (11) and a plurality of function keys (12), and internally provided with a heartbeat measuring circuit (2) and a body fat measuring circuit (3). Two separated measuring contacts are provided on the watch body (1) at predetermined positions to electrically connect to the body fat measuring circuit (3), and an infrared emitter (21) and infrared receiver (22) are provided on the watch body (1) to electrically connect to the heartbeat measuring circuit (2). The wrist watch may be conveniently worn on user's wrist without occupying extra space while it enables the user to monitor health conditions at any time and place.

## Description

### FIELD OF THE INVENTION

The present invention relates to a wrist watch with health condition monitoring mechanism, and more particularly to a common-sized wrist watch having three different functions, including time display, heartbeat counting, and body fat measuring, integrated therein to enable a user to conveniently monitor health conditions at any time and place.

### BACKGROUND OF THE INVENTION

A lot of people living in abundance have the problem of overweight due to lack of exercise. Under this condition, it is very important for people, particularly fat people, to frequently measure their heartbeats and body fat as a means to monitor their health conditions and body functions and avoid various kinds of fat-related illness.

Thanks to the advanced electronic technologies, there are a variety of electronic heartbeat and body fat measuring means developed and available in the market.

A common disadvantage among the currently available heartbeat and body fat measuring means is they all have large volume and are structurally independent of one another, and are therefore space-occupying and inconvenient for carrying at the same time.

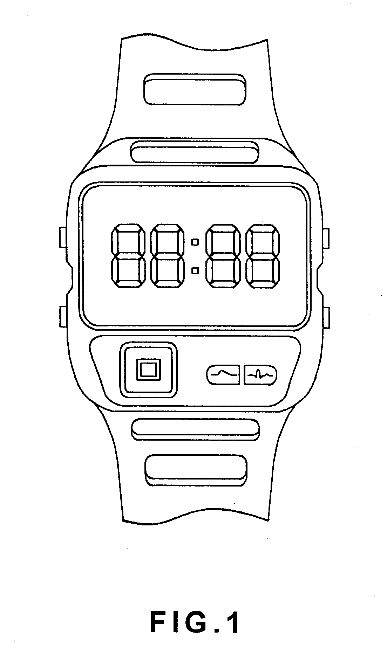

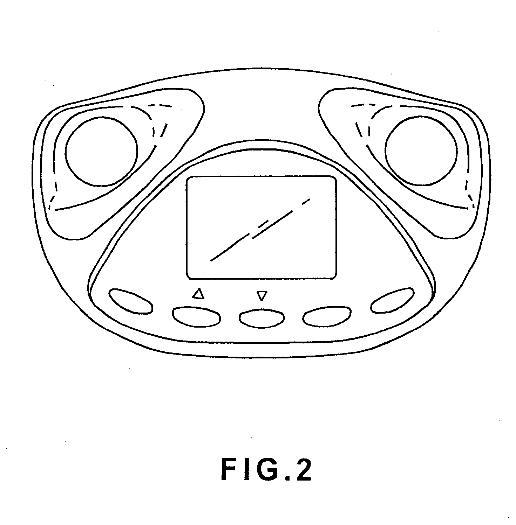

Fig. 1 shows a conventional wrist watch that is incorporated with a heartbeat gauge but not a body fat gauge, and therefore not sufficient in terms of self-monitoring of a user's health conditions. Conventional body fat gauges may be generally divided into two types, a first one of which is incorporated in a scale and includes two metal electrodes mounted on a top panel of the scale. To use the scale to measure body fat, a user has to take off shoes and socks and stands on the two metal electrodes, so as to measure the body weight and body fat at the same time. This type of scale-incorporated body fat gauge has a large volume and is not convenient for carrying. It is usually positioned in a bathroom or living room and a user would usually take the measurements after bathing before taking on shoes and socks. Another type of conventional body fat gauge is a pocket-sized product developed for convenient carrying thereof. As can be seen from Fig. 2, this pocket-sized body fat gauge includes two metal contact electrodes arranged at two lateral sides of the gauge. To measure body fat percentage, a user may hold the gauge and touches the two metal contact electrodes with two thumbs. This pocket-sized body fat gauge still has a considerable volume and requires a pocket to hold it, and provides only one single function. It does not display time and could not be used to count heartbeats.

It is therefore tried by the inventor to develop a wrist watch with heartbeat and body fat measuring mechanism for users to conveniently monitor their health conditions at any time and place with only one single piece of wrist watch.

### SUMMARY OF THE INVENTION

A primary object of the present invention is to integrate three different functions, including time display, heartbeat counting, and body fat measuring, in a common-sized wrist watch, so that a user may conveniently wear the wrist watch to monitor health conditions at any time and place simply by pushing buttons provided on the watch to switch the watch among different functional modes.

To achieve the above and other objects, the present invention provides a wrist watch with heartbeat and body fat measuring mechanism. The wrist watch mainly includes a watch body being externally provided with a liquid crystal display and a plurality of function keys, and internally provided with a heartbeat measuring circuit and a body fat measuring circuit. Two separated measuring contacts are provided on the watch body at predetermined positions to electrically connect to the body fat measuring circuit, and a detecting module including infrared emitter and receiver is provided on the watch body to electrically connect to the heartbeat measuring circuit. The wrist watch may be conveniently worn on a user' s wrist without occupying extra space while it enables the user to monitor health conditions at any time and place.

### BRIEF DESCRIPTION OF THE DRAWINGS

The structure and the technical means adopted by the present invention to achieve the above and other obj ects can be best understood by referring to the following detailed description of the preferred embodiments and the accompanying drawings, wherein
Fig.1 shows a conventional wrist watch having a heartbeat gauge incorporated therewith;
Fig. 2 shows a conventional pocket-sizedbody fat gauge;
Fig. 3 is a front view of a wrist watch with heartbeat and body fat measuring mechanism according to an embodiment of the present invention;
Fig. 4 shows the use of the present invention to measure a user' s body fat by touching two contacts on the wrist watch;
Fig. 5 is a side view of Fig. 4; and
Fig. 6 shows the use of the present invention to count a user's heartbeats.

### DETAILED DESCRIPTION OF THE PREFERRED EMBODIMENTS

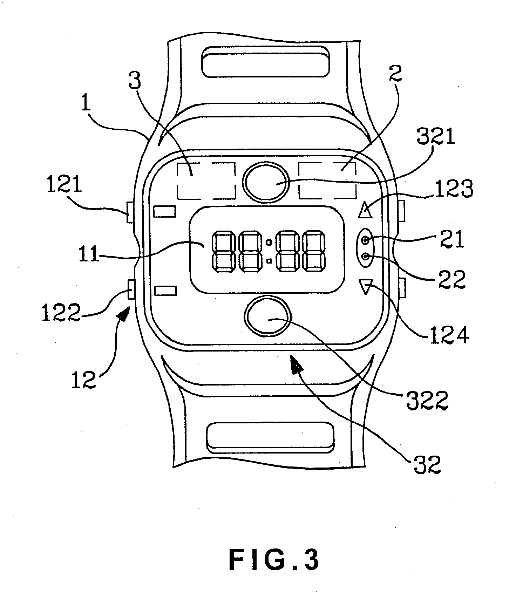

Please refer to Fig. 3 that is a front view of a wrist watch with heartbeat and body fat measuring mechanism according to an embodiment of the present invention. As shown, the wrist watch mainly includes a watch body 1, on a top surface of which a liquid crystal display (LCD) 11 and a plurality of function keys 12 areprovided. The watch body 1 is internally provided with a heartbeat measuring circuit 2 and a body fat measuring circuit 3. The watch body 1 is also externally provided at predetermined positions with two separated measuring contacts 31, 32, which are electrically connected to the body fat measuring circuit 3.

One of the two measuring contacts, which is the contact 31 in the illustrated embodiment, is located at a bottom side of the watch body 1 for directly bearing against a user's skin. The other measuring contact 32 is provided at the top surface of the watch body 1 and consists of two metal contacts 321, 322 separately located above and below the LCD 11. The function keys 12 on the watch body 1 include a SET key 121, a MODE key 122, and ▲ (UP) and ▼ (DOWN) adjusting buttons 123, 124. A detecting module consisting of an infrared emitter 21 and an infrared receiver 22 is also provided on the watch body 1 to electrically connect to the heartbeat measuring circuit 2.

Unlike the conventional pocket-sized body fat gauge of Fig. 2 that has metal contact electrodes located at two lateral sides of the device, the wrist watch of the present invention has measuring contacts 31, 32 separately located at top and bottom sides of the watch body 1. This arrangement advantageously allows the wrist watch of the present invention to be conveniently worn on a user' s wrist without occupying extra space. In brief, the present invention effectively integrates three different measuring devices for time, heartbeat, and body fat into one common-sized wrist watch. The wrist watch of the present invention not only displays time but also serves as a convenient means for effectively measuring the user's body fat and heartbeats, so that the user may correctly control the health condition at any time and place.

As can be seen from Fig. 3, the function keys 12 include four push buttons in two groups separately located at two lateral sides of the watch body 1. The MODE key 122 allows a user to select and switch among different functions. The watch body 1 normally functions like a timepiece. When the MODE key 122 is pushed once, the watch body 1 is switched to function as a heartbeat counter. When the MODE key 122 is pushed again, the watch body 1 is switched to function as a body fat gauge. And, when the MODE key 122 is pushed once more, the watch body 1 returns to its time display function.

The SET key 121 is used to set different functions for different modes. When the watch body 1 is in the time mode, time setting may be achieved by pushing the SET key 121. When the watch body 1 is in the heartbeat counter mode, display of maximum, minimum, or average heartbeat value may be set via the SET key 121. When the watch body 1 is in the body fat gauge mode, parameters, such as height, weight, age, sex, etc., may be set via the SET key 121. The ▲ (UP) and **▼** (DOWN) adjusting buttons 123, 124 are used to set higher or lower value for each setting.

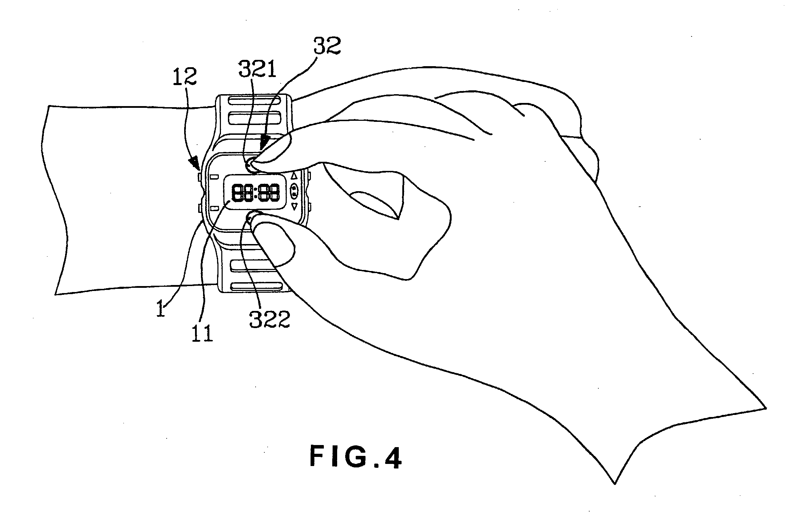

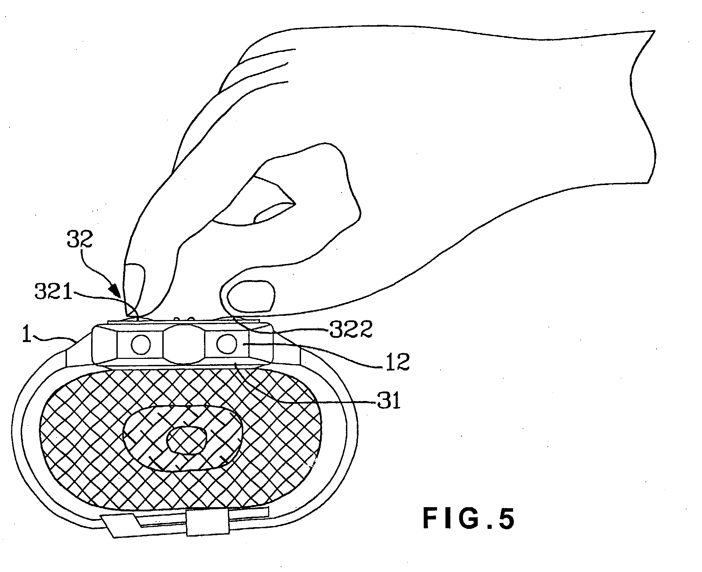

Figs. 4 and 5 shows the use of the present invention to measure the user's body fat. In this case, since the measuring contact 31 at the bottom side of the watch body 1 to serve as an electrode is in natural contact with the user's wrist, the user needs only to touch the two metal contacts 321, 322 of the other measuring contact 32 serving as another electrode with thumb and index finger of the other hand. In this manner, the user's two hands together form a close circuit. Since the body fat has impedance larger than that of muscles, it is possible to calculate the percentage of body fat in the user's body according to the magnitude of impedance displayed on the LCD 11.

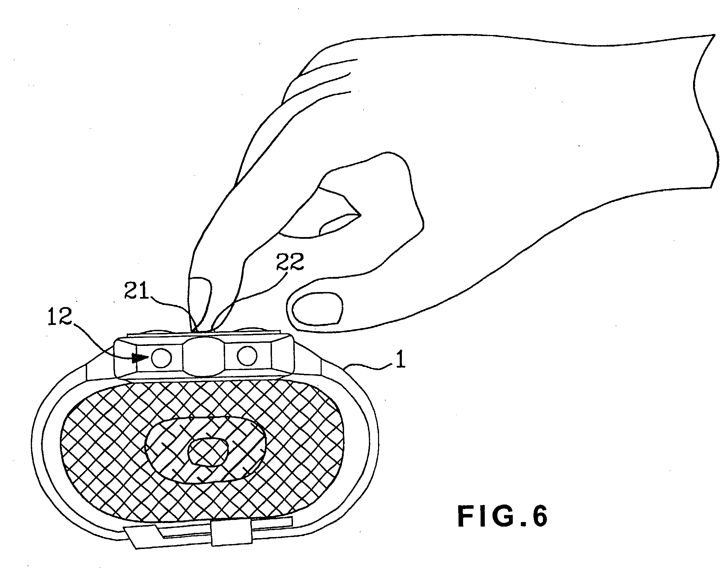

Please refer to Figs. 3 and 6 at the same time. As mentioned above, there is a detecting module consisting of an infrared emitter 21 and an infrared receiver 22 provided on the watch body 1 at predetermined positions. To measure the heartbeats with the wrist watch of the present invention, a user needs only to touch the detecting module with one finger of the other hand. The user's blood density in vessels changes at systole and diastole, resulting in changes in the reflected infrared signal of the detecting module. By sending the reflected infrared signals to an input of an internal central processing unit (CPU) (not shown) in the wrist watch of the present invention, the user' s heartbeats per minute may be calculated.

The present invention has been described with a preferred embodiment thereof and it is understood that many changes and modifications in the described embodiment can be carried out without departing from the scope and the spirit of the invention as defined by the appended claims.

## Claims

1. A wrist watch with heartbeat and body fat measuring mechanism, comprising a watch body (1), on a top surface of which a liquid crystal display (LCD) (11) and a plurality of function keys (12) are provided; said watch body (1) being internally provided with a heartbeat measuring circuit (2) and a body fat measuring circuit (3), and externally provided at predetermined positions with two separated measuring contacts (31, 32) that are electrically connected to said body fat measuring circuit (3), whereby three different functions, including time display, body fat measuring, and heartbeat counting, are integrated in said watch body for a user to conveniently monitor his or her health conditions.

2. The wrist watch with heartbeat and body fat measuring mechanism as claimed in claim 1, wherein one (31) of said two separated measuring contacts is located at a bottom side of said watch body to directly bear against a user's skin, and the other one (32) of said two separated measuring contacts is located at a top side of said watch body and includes two electrically connected metal contacts (321, 322) separately located above and below said LCD.

3. The wrist watch with heartbeat and body fat measuring mechanism as claimed in claim 1, wherein said a plurality of function keys (12) include a MODE key (122) for switching said watch body (1) among different modes and selecting a desired mode, a SET key (121) for setting different functions for selected modes, and a pair of ▲(UP) and ▼(DOWN) adjusting buttons (123, 124) for setting higher or lower value for each setting made via said SET key (121).

4. The wrist watch with heartbeat and body fat measuring mechanism as claimed in claim 1, wherein said watch body (1) is further provided with a detecting module including an infrared emitter (21) and an infrared receiver (22) electrically connected to said heartbeat measuring circuit (2).
